Europäisches Patentamt

(19) European Patent Office    (11) Publication number:    **0 005 074**

Office européen des brevets    **A1**

(12) **EUROPEAN PATENT APPLICATION**

(21) Application number: **79300682.6**    (51) Int. Cl.²: **A 61 K 31/48**

(22) Date of filing: **24.04.79**

(30) Priority: **24.04.78 US 898741**

(43) Date of publication of application:
**31.10.79 Bulletin 79 '22**

(84) Designated Contracting States:
**BE CH DE FR GB IT LU NL SE**

(71) Applicant: **MASSACHUSETTS INSTITUTE OF TECHNOLOGY**
**77 Massachusetts Avenue**
**Cambridge Massachusetts 02139(US)**

(72) Inventor: **Fernstrom, John D**
**65 East India Row**
**Boston Massachusetts(US)**

(72) Inventor: **Sved, Alan F.**
**1401 Commonwealth Avenue**
**Brighton Massachusetts(US)**

(74) Representative: **Harvey, David G. et al,**
**Graham Watt & Co. Riverhead**
**Sevenoaks Kent TN13 2BN(GB)**

(54) **A material and composition for reducing blood pressure.**

㊼ A material and composition for reducing blood pressure are provided. The blood pressure in animals is reduced by administering lergotrile, viz. 2-chloro-6-methylergoline-8β-acetonitrile or pharmaceutically acceptable salts thereof either alone or in combination with a substance which is known to reduce blood pressure. A composition is provided comprising a unit dosage form of lergotrile and a substance which is known to reduce blood pressure. For lergotrile, any of its physiologically- and pharmacologically-active metabolites in the body can be substituted.

**EP 0 005 074 A1**

## "A MATERIAL AND COMPOSITION FOR REDUCING BLOOD PRESSURE."

The present invention relates to material for use in reducing blood pressure which avoids or reduces the release of serum prolactin. The invention also relates to a pharmaceutical composition comprising such a material.

At the present time, the most widely used medicaments for lowering blood pressure, such as $\alpha$-methyl dopa (Aldomet), 11,17$\alpha$-dimethoxy-18$\beta$- [(3,4,5 -trimethoxy-benzoyl)oxyl] -3$\beta$,20$\alpha$-yohimban-16$\beta$-carboxylic acid methyl ester (Reserpine) or 2-(2,6-dichlorophenylamino) 2-imidazoline hydrochloride (Clonidine hydrochloride), also stimulate the secretion of prolactin in humans. This side effect is potentially objectionable, inasmuch as in recent years, a category of breast carcinoma has been described the development of which is enhanced by high circulating levels of prolactin. It has been shown in experimental animals that Reserpine, which is used commonly to treat hypertension and release prolactin, also increases the incidences of breast cancer.

The invention is intended to remedy the shortcomings found with the commonly used medicaments and has for an object the provision of an otherwise innocuous means for reducing hypertension in animals and which could serve either as a substitute for or an adjunct to present means for reducing hypertensions in order to eliminate or substantially reduce the problems of increased prolactin secretion which accompany the administration of presently available drugs.

We have found that the compound, 2-chloro-6-methylergoline-8$\beta$-acetonitrile (lergotrile) or

0005074

pharmaceutically acceptable salts thereof reduces hypertension when administered to animals without effecting increased secretion of prolactin.

The invention provides a material for use in reducing blood pressure in the human or animal body characterised by lergotrile and/or a pharmaceutically acceptable salt of lergotrile.

This material may be associated with a blood pressure reducing agent selected from alpha-methyl dopa; 11,17alpha-dimethoxy-18Beta-[(3,4,5-trimethoxybenzoyl)oxy] -3Beta,20alpha-yohimban-16Beta-carboxylic acid methyl ester; 2-(2,6-dichlorophenylamino) 2-imidazoline and pharmaceutically acceptable salts thereof and mixtures thereof.

The said blood pressure reducing agent can be provided separately or in admixture with the said material.

The invention also provides a composition in pharmaceutical dosage form comprising material according to the invention, and if desired the composition can contain one or more of the aforementioned blood pressure reducing agents.

The invention also provides a pharmaceutical composition comprising an excipient and material according to the invention.

The invention also provides a pharmaceutical composition comprising (a) lergotrile and/or a pharmaceutically acceptable salt of lergotrile and (b) a blood pressure reducing agent selected from alpha-methyl dopa; 11, 17alpha-dimethoxy-18Beta-[(3,4,5 -trimethoxy- benzoyl)oxy] -3Beta,20alpha-yohimban-16Beta-carboxylic acid methyl ester; 2-(2,6-dichlorophenylamino) 2-imidazoline pharmaceutically acceptable salts thereof and mixtures thereof.

Material in accordance with the invention can be administered intraperitoneally, subcutaneously, intramuscularly or orally.

The term "lergotrile" as used in this specification and the claims means lergotrile per se as well as its physiologically and pharmacologically active metabolites in the body.

The following description of the practice of the invention is given by way of example only and not by way of limitation of the invention.

The compound 2-chloro-6-methylergoline-8$\beta$-acetonitrile (lergotrile) or its pharmaceutically acceptable salts are available from Eli Lilly and Company, Indianapolis, Indiana. Prior to the present invention, lergotrile has been administered to humans to suppress the secretion of prolactin by the pituitary gland, and to patients suffering from Parkinson's disease, to attempt to reduce involuntary tremor. It is surprising that lergotrile also reduces blood pressure since it is a known agent for reducing serum prolactin levels; the commonly used blood pressure reducing agents in contrast effect increased secretion of serum prolactin levels.

While the mechanism by which lergotrile produces reduced blood pressure has not yet been determined, it is believed that the mechanism may be different than that of Aldomet, Clonidine Hydrochloride or Reserpine since it actually reduces prolactin secretion rather than increasing it. In addition, unlike Aldomet, lergotrile reduces blood pressure in animals having normal blood pressure.

The administration of the present compositions can be effected orally, intraperitoneally, subcutaneously, intraveneously or intramuscularly. Conveniently, the compositions are admixed or dissolved in any innocuous excipient such as water or sterile saline solution or are provided in tablet or powder form containing the usual solid diluents or carriers. When producing a lowering of blood pressure, the compositions employed in the present invention are administered in concentrations to avoid undesirable side effects. The compound, lergotrile, is employed in dosages sufficient to effect lowering of blood pressure while minimizing the possibility of producing undesirable side effects such as orthostatic hypotension. In humans, useful dosages are preferably from 0.01 mg/kg to 1 mg/kg, and more preferably from 0.1 mg/kg to 0.5 mg/kg. Dosages below 0.01 mg/kg body weight do not produce significant lowering of blood pressure while concentrations above 1.0 mg/kg body weight do not produce significant additional lowering of blood pressure and may produce undesirable side effects. Lowering of blood pressure is produced for about 4.5 hours.

It has also been found that the co-administration of lergotrile and a compound selected from Aldomet, Reserpine and Clonidine or their pharmaceutically acceptable salts (which dissociate in vivo to produce one of these compounds) produces an additive effect of lowering blood pressure. In order to obtain this additive effect, concentrations of lergotrile are between 0.01 and 0.5 mg/kg body weight compounded with the main antihypertensive agent. In co-administration compositions, Reserpine is employed in amounts between .007 and .0035 mg/kg, Aldomet in amounts between 1.5 mg/kg and 30 mg/kg and Clonidine in amounts between .0014 mg/kg and .035 mg/kg.

EXAMPLE I.

This example illustrates that lergotrile alone produces a lowering blood pressure in animals having either normal or high blood pressure.

Male SHR (Spontaneously Hypertensive Rats), rats of the Okamoto Strain, and normotensive Sprague-Dawley rats (Charles River Breeding Laboratories) weighing between 350 and 450 grams were used in these experiments. The animals were housed in pairs and given ad libitum access to food and water. Blood pressures were determined using the tail vein plethysmography technique: Rats were warmed at $38^{o}C$ for 15 minutes prior to each measurement to dilate the tail vessels. The tail cuff then was placed around the tail with the pressure sensor attached. Eight readings were taken rapidly for each timepoint studied. The pressure monitoring system utilized was a Narco Biosystems Pneumatic Pulse Transducer. All animals used in the studies had their blood pressures taken several times, on separate days, to acclimate them to the procedure and thus minimize falsely high blood pressures due to the stress of an unfamiliar procedure. Experiments were performed in the morning or early afternoon. Lergotrile mesylate in varying amounts was injected intraperitoneally in a water solution and control animals received water alone. Blood pressure measurements were made prior to and after drug administration. At each timepoint, the animals were warmed and then eight successive pressure readings were taken rapidly for each animal. In the dose-response experiments, blood pressures

were taken one hour after drug administration, as well as shortly before drug injection. One hour post injection was determined to be the timepoint at which the maximal lowering effect was observed. The results are shown in Tables I, II and III.

## TABLE I

BLOOD PRESSURE LOWERING IN NORMOTENSIVE RATS

| Lergotrile Dosage, mg/kg. | Blood Pressure Change, mm Hg I Hour after Administration |
|---|---|
| 0 | — |
| 0.1 | -11.2 |
| 0.5 | -16.2 |
| 1.0 | -28.7 |

## TABLE II

BLOOD PRESSURE LOWERING IN SHR RATS

| Lergotrile Dosage, mg/kg. | Blood Pressure Change, mm Hg I Hour after Administration |
|---|---|
| 0 | — |
| 0.05 | -28 |
| 0.1 | -42 |
| 0.25 | -65.5 |
| 0.5 | -70.5 |
| 1.0 | -76 |

## TABLE III

| Time after<br>Lergotrile Administration | Blood Pressure<br>Change, mm Hg |
|---|---|
| 0 | 0 |
| 1.5 | -57 |
| 3.0 | -30 |
| 4.5 | -12 |

Dose employed:   0.5 mg/kg

## EXAMPLE II.

This example shows that the administration of Aldomet and lergotrile has an additive effect in lowering blood pressure.

The procedures for measuring blood pressures herein are set forth in Example I. Male SHR rats were used in this experiment. Blood pressures were taken just prior to administration. In a first administrative step, lergotrile (0.1 mg/kg) or water (as controls) was administered to the rats. Blood pressures were taken 1 hour later. Aldomet (50 mg/kg) then was administered to both groups. Blood pressures then were taken 1.5 hours after the Aldomet was administered. The results are shown in Table IV.

## TABLE IV

| Time | Water & Aldomet<br>Blood Pressure, mm Hg | Lergotrile & Aldomet<br>Blood Pressure, mm Hg |
|---|---|---|
| - 1 hour | 212 | 206 |
| 0 | 210 | 157 |
| + 1.5 hours | 176 | 131 |

0005074

As shown in Table IV, lergotrile by itself had a significant lowering effect on blood pressure. The administration of Aldomet had a significant effect of lowering blood pressure in both groups of animals. Thus, lergotrile does not block the action of Aldomet in reducing blood pressure. Furthermore, this Aldomet and lergotrile treatment has the desirable effect of lowering serum prolactin levels. Experiments conducted by administering lergotrile and Aldomet simultaneously also show the additive blood pressure lowering effect:

### TABLE V

| Time | Aldomet (25 mg/kg) Blood Pressure, mm Hg | Aldomet (25 mg/kg) Lergotrile (0.1 mg/kg) Blood Pressure, mm Hg |
|---|---|---|
| 0 | - | - |
| 1.5 | -25 | -56 |
| 3.0 | -38 | -77 |
| 4.5 | -29 | -27 |

Claims:

1.    A material for use in reducing blood pressure in the human or animal body characterised by lergotrile and/or a pharmaceutically acceptable salt of lergotrile.

2.    A material according to claim 1, characterised by association with a blood pressure reducing agent selected from alpha-methyl dopa; 11,17 alpha-dimethoxy-18Beta- [(3,4,5-trimethoxybenzoyl)oxy] -3Beta,20alpha-yohimban-16Beta-carboxylic acid methyl ester; 2-(2,6-dichlorophenylamino) 2-imidazoline, and pharmaceutically acceptable salts thereof and mixtures thereof.

3.    A composition in pharmaceutical dosage form comprising material according to claim 1 or claim 2.

4.    A composition in pharmaceutical dosage form comprising material according to claim 2 suitable for administration in a dosage of lergotrile between 0.01 and 0.5 mg/kg.

5.    A pharmaceutical composition comprising an excipient and material according to claim 1 or claim 2.

6.    A pharmaceutical composition according to claim 5 suitable for administration in a dosage of lergotrile between 0.01 and 1 mg/kg.

7.    A pharmaceutical composition comprising (a) lergotrile and/or a pharmaceutically acceptable salt of lergotrile and (b) a blood pressure reducing agent selected from alpha-methyl dopa; 11,17alpha-dimethoxy-

-18Beta-[(3,4,5-trimethoxybenzoyl)oxy] -3Beta, 20alpha-yohimban-16Beta-carboxylic acid methyl ester; 2-(2,6-dichlorophenylamino) 2-imidazoline, pharmaceutically acceptable salts thereof and mixtures thereof.

8.    A composition according to claim 7, in a form suitable for administration in a dosage of component (a) between 0.01 and 0.5 mg/kg.

0005074

Application number

# EUROPEAN SEARCH REPORT

European Patent Office

EP 79 30 0682

| Category | Citation of document with indication, where appropriate, of relevant passages | Relevant to claim |
|---|---|---|
| A | US - A - 3 904 757 (IRWIN H. SLATER)<br><br>* claim 1 *<br><br>------- | 1-8 |

**DOCUMENTS CONSIDERED TO BE RELEVANT**

**CLASSIFICATION OF THE APPLICATION (Int. Cl.²)**

A 61 K 31/48

**TECHNICAL FIELDS SEARCHED (Int.Cl.²)**

A 61 K 31/48
C 07 D 457/02

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 25-07-1979 | BRINKMANN |

EPO Form 1503.1   06.78